(19) **Europäisches Patentamt / European Patent Office / Office européen des brevets**

(11) **EP 2 436 274 B1**

(12) **EUROPEAN PATENT SPECIFICATION**

(45) Date of publication and mention
of the grant of the patent:
**28.12.2016 Bulletin 2016/52**

(51) Int Cl.:
***A61K 35/20*** (2006.01)

(21) Application number: **11009093.3**

(22) Date of filing: **31.03.2005**

(54) **Antibacterial compositions**

Antibakterielle Zusammensetzungen

Compositions antibactériennes

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **31.03.2004 JP 2004107031**

(43) Date of publication of application:
**04.04.2012 Bulletin 2012/14**

(62) Document number(s) of the earlier application(s) in accordance with Art. 76 EPC:
**05727707.1 / 1 743 647**

(73) Proprietor: **Meiji Co., Ltd.**
**Tokyo 136-8908 (JP)**

(72) Inventors:
 • **Kume, Hisae**
   **Odawara-shi**
   **Kanagawa 2500862 (JP)**

 • **Sasaki, Hajime**
   **Odawara-shi**
   **Kanagawa 2500862 (JP)**

(74) Representative: **Grünecker Patent- und Rechtsanwälte**
**PartG mbB**
**Leopoldstraße 4**
**80802 München (DE)**

(56) References cited:
**EP-A1- 1 048 690      EP-A2- 0 233 565**
**US-A- 3 963 836**

 • **KAZUHIRO OKUMA ET AL: "Development of Indigestible Dextrin", JOURNAL OF APPLIED GLYCOSCIENCE, vol. 53, no. 1, 1 January 2006 (2006-01-01), pages 65-69, XP055295076, JP ISSN: 1344-7882, DOI: 10.5458/jag.53.65**

Remarks:
The file contains technical information submitted after the application was filed and not included in this specification

**Description**

Technical Field

The present invention relates to compositions having an antibacterial activity.

Background Art

Liquid diets are widely used in hospitals and institutions for the aged, for individuals with swallowing disorders or those having difficulties with oral nutritional intake. Liquid diets are administered through a catheter or tube inserted into the body, for example, the nasal cavity or stomach (by PEG (Percutaneous Endoscopic Gastrostomy)). Generally, those taking liquid diets are aged individuals or patients with diseases of some kind, who are often immunocompromised and are thus susceptible to pathogens.

Once an infection occurs in such an institution with many people who can get easily infected, it may develop into a hospital-acquired infection or an institutional infection through health care personnel. Although there is an increasing awareness of measurements to prevent hospital-acquired infections, this issue is gaining more and more attention with the increasing number of aged individuals and the emergence of resistant bacteria. Until the 1970's, *Pseudomonas aeruginosa* was the predominant bacteria causing hospital-acquired infections in Japan, but from 1980 onwards, MRSA has become the leading cause. Since MRSA exhibits resistance against antibiotics, it is a type of pathogenic bacteria for which countermeasures are particularly required.

If a liquid diet can have antibacterial effects, it may be used as a means of protecting easily infected people from infection; however, reports on the antibacterial effect of liquid diets are yet to be seen.

[Non-patent Document 1] Goepfert, J. M., Chung, K. C., Behavior of salmonellae in sliced luncheon meats, Appl. Microbiol., 19, 190-192, 1970.
[Non-patent Document 2] Nakae, E., Science of Milk, Meat, and Eggs, Kougaku Shuppan, p132-137, 1996.
[Non-patent Document 3] Kawai Y, Saito, T., Kitazawa, H., and Itoh T., Biosci. Biotech. Biochem.62, 22438-40, 1998.
[Non-patent Document 4] Shah, N. P.: Some beneficial effects of probiotic bacteria, Bioscience Microflora, 19, 2, 99-106, 2000.

Disclosure of the Invention

An objective of the present invention is to provide compositions having an antibacterial activity, which may be utilized as a means of protection against infection.

To solve the above-mentioned objective, the present inventors focused on acidic liquid diets. Liquid diets are usually prepared to have a neutral pH. However, the acidic liquid diets focused on by the present inventors were acidic because they contained a fermented dairy product as protein source. When the present inventors examined the antibacterial effect of the acidic liquid diets, an excellent antibacterial activity against Gram-negative bacteria such as E. coli and *Pseudomonas aeruginosa* was seen. Furthermore, when the effect against Gram-positive bacteria was examined, surprisingly, the acidic liquid diets demonstrated an antibacterial effect remarkably higher than that of the basic ingredient, the fermented dairy product.

It is well known that microorganisms are sensitive to acidic environments. The inhibitory effects of acids on the growth of microorganisms depend on the hydrogen ion concentration. At the same pH, an organic acid such as lactic acid or acetic acid, is said to demonstrate a higher antibacterial effect than an inorganic acid, for example, hydrochloric acid or sulfuric acid. The lowest pH at which bacteria belonging to the genus of *Salmonella* can grow has been reported to be pH5.4 in acetic acid or propionic acid, and pH4.4 in lactic acid (Non-patent Document 1).

The antibacterial effect of fermented milk such as yogurt is already known. Antibacterial substances produced by *Lactobacilli* in addition to lactic acid, include a group of low-molecular weight compounds produced in small amounts as byproducts, such as volatile fatty acids (acetic acid, formic acid), hydrogen peroxide, β-hydroxypropionaldehyde (reuterin), and 2-pyrrolidone-5-carboxylic acid (Non-patent Document 2). Known bacteriocins produced by *Lactobacilli* include nisin produced by *Lactococcus lactis* subsp. *lactis,* and gassericin A produced by *Lactobacillus gasseri* LA-39 (Non-patent Document 3). Known probiotic effects of *Lactobacilli* are as follows: suppressive effects on adhesion of pathogenic bacteria to the intestinal epithelium, therapeutic/prophylactic effects on infection by producing antibacterial substances, stimulative effects on host immunogenic potential by activating macrophages and maintaining a healthy intestinal environment, and prevention of diarrhea/constipation (Non-patent Document 4).

However, even with the already known knowledge described above, the finding that the antibacterial action of a composition prepared from a fermented dairy product is remarkably higher than that of the basic ingredient was totally unexpected. In other words, the present invention relates to compositions having antibacterial effect, and specifically provides the following:

[1] an antibacterial composition, wherein the composition is

(a) prepared using a fermented dairy product,
(b) comprises carbohydrates, proteins, and fats, and
(c) has a pH of 4.6 or less,

and wherein the energy ratio of carbohydrates, proteins, and fats is 50% to 70%, 4% to 25%, and 20% to 30%, respectively;

[2] the antibacterial composition of [1], wherein the fermented dairy product is fermented milk and/or a natural cheese;

[3] the antibacterial composition of [1] or [2], wherein the amount of lactic acid in 100 mL of the composition is 200 mg or more;

[4] the antibacterial composition of [1] or [2], wherein the amount of lactic acid in 100 mL of the composition is 300 mg or more;

[5] the antibacterial composition of any one of [1] to [4], which comprises the constituent combination of any one of Table 1, 3, or 5;

[6] a method for producing an antibacterial composition, wherein the method comprises:

(a) using a fermented dairy product as an ingredient,
(b) solubilizing the fermented dairy product in advance,
(c) mixing the fermented product with other ingredients in a composition after adding a stabilizer, wherein the composition comprises carbohydrates, proteins, fats, vitamins and minerals, and
(d) homogenizing and sterilizing the mixture;

[7] the method for producing the antibacterial composition of [6], wherein the fermented dairy product is fermented milk and/or a natural cheese;

[8] the method for producing the antibacterial composition of [6] or [7], wherein the method comprises preparing the composition such that the proteins derived from the fermented dairy product account for 30 weight % or more of the proteins in the composition; and

[9] the method for producing the antibacterial composition of [6] or [7], wherein the method comprises preparing the composition such that the proteins derived from the fermented dairy product account for 70 weight % or more of the proteins in the composition.

## Brief Description of the Drawings

Fig. 1 shows the viable bacterial cell count per 1 mL of samples collected after adding E. *coli* or *Pseudomonas aeruginosa* to an acidic liquid diet, a neutral liquid diet, and a liquid fermented milk preparation, and culturing these for 3, 6, and 24 hours.

Fig. 2 shows the viable bacterial cell count per 1 mL of samples collected after adding *Streptococcus mutans, Staphylococcus aureus* subsp. *aureus,* or MRSA to the acidic liquid diet, neutral liquid diet, and liquid fermented milk preparation, and culturing these for 3, 6, and 24 hours.

Fig. 3 shows the viable bacterial cell count per 1 mL of samples collected after adding E. *coli, Pseudomonas aeruginosa, Staphylococcus aureus* subsp. *aureus,* or MRSA to neutral liquid diets adjusted to pH4 by adding lactic acid, acetic acid, or citric acid, and culturing these for 3, 6, and 24 hours.

Fig. 4 shows the viable bacterial cell count per 1 mL of samples collected after adding E. *coli, Pseudomonas aeruginosa, Staphylococcus aureus* subsp. *aureus,* or MRSA to neutral liquid diets adjusted to pH 4 to 6.1 by adding 0.125, 0.25, 0.5, or 1 mL of lactic acid, and culturing these for 3, 6, and 24 hours.

Fig. 5 shows the viable bacterial cell count per 1 mL of samples collected after adding *Helicobacter pylori* or *Clostridium difficile* to the acidic liquid diet, neutral liquid diet, and liquid fermented milk preparation, and culturing these for 3, 6, and 24 hours.

Fig. 6 shows the viable bacterial cell count per 1 mL of samples collected after adding *Clostridium difficile* to a neutral liquid diets adjusted to pH 4 to 6.1 by adding 0.125 mL or 1mL of lactic acid, and culturing these for 3, 6, and 24 hours.

Fig. 7 shows the viable bacterial cell count per 1 mL of samples collected after adding MRSA or *Clostridium difficile* to the acidic liquid diet, acidic liquid diet 2, neutral liquid diet, and liquid fermented milk preparation, and culturing these for 3, 6, and 24 hours.

Best Mode for Carrying Out the Invention

The present invention provides antibacterial compositions (hereinafter referred to as compositions of the present invention). The present inventors confirmed antibacterial effects of acidic liquid diets; in particular, an outstanding antibacterial effect against Gram positive bacteria was discovered. Compositions of the present invention are acidic. Their pH is preferably 4.6 or less, more preferably less than 4.2, and most preferably less than 4.0.

Compositions of the present invention comprise carbohydrates, proteins, fats, vitamins, and minerals. Carbohydrates that may be used in the compositions of the present invention are not limited as long as they are edible. Examples include honey, dextrin, sucrose, palatinose, glucose, granulated sugar, fructose, starch syrup candy, and sugar alcohols (sorbitol, xylitol, maltitol, and such). Proteins that may be used in the compositions of the present invention are also not limited as long as they are edible. Examples include proteins derived from milk, such as casein and whey protein, soy protein, and hydrolysates of these proteins produced by using neutrase, alkalase, or animal-derived enzymes such as trypsin and pepsin. Fats that may be included in the compositions of the present invention are also not limited as long as they are edible. Examples include sunflower oil, rapeseed oil, olive oil, and safflower oil containing monovalent saturated fatty acids and polyvalent unsaturated fatty acids, as well as various types of oils and fats of plant origin such as corn oil, soybean oil, palm oil, perilla oil (from *Perilla frutescens* (L.) Britt. var. *frutescens, or Perilla frutescens* var. *crispa*), linseed oil, *Oenothera tetraptera* oil, palm kernel oil, and coconut oil, middle-chain fatty acids, EPA, DHA, soybean derived phospholipids, and milk derived phospholipids; preferably, one or more types of oils and fats of plant origin are included. Furthermore, the percentage of polyvalent unsaturated fatty acids in the present invention, which contains two or more double bonds is 10% or more, preferably 20% or more, or more preferably 25% or more of the fatty acid composition in oils and fats. Vitamins that may be included in the compositions of the present invention are not particularly limited, and may be any one of known vitamins. Minerals that may be included in the compositions of the present invention may be any of those generally added to foods, and examples include potassium phosphate, potassium carbonate, potassium chloride, sodium chloride, calcium lactate, calcium gluconate, calcium pantothenate, calcium caseinate, magnesium chloride, citric acid, ferrous sulfate, sodium hydrogen carbonate, sodium pyrophosphate, and sodium ascorbate. Other components may be included in all of the carbohydrates, proteins, fats, vitamins, and minerals, without being restricted to the specific examples described above, as long as the antibacterial activity of the composition is maintained.

The energy ratio of the above-mentioned carbohydrates, proteins, and fats in the compositions of the present invention are 50% to 70%, 4% to 25%, and 20% to 30%, respectively. Dietary fibers are not included in carbohydrates when calculating the energy ratio. Energy ratios can be determined as follows:

$$\text{Protein energy ratio} = \text{protein amount (g)} \times 4 \text{ (kcal/g)} \div \text{total food energy (kcal)} \times 100$$

$$\text{Fat energy ratio} = \text{fat amount (g)} \times 9 \text{ (kcal/g)} \div \text{total food energy (kcal)} \times 100$$

$$\text{Carbohydrate energy ratio} = \text{carbohydrate amount (g)} \times 4 \text{ (kcal/g)} \div \text{total food energy (kcal)} \times 100, \text{ or}$$

$$\text{Carbohydrate energy ratio} = \{\text{total food energy (kcal)} - (\text{protein energy (kcal)} + \text{fat energy (kcal)} + \text{dietary fiber energy (kcal)})\} \div \text{total food energy (kcal)} \times 100$$

The energy of dietary fibers can be calculated based on the Standard Tables of Food Composition in Japan (published by the Ministry of Finance, National Printing Bureau) or the Energy Conversion Coefficient (Ministry of Health, Labor and Welfare, Notice #98; 2001).

The amount of carbohydrates, proteins, and fats can be measured by methods well known to those skilled in the art, preferably according to the Standard Tables of Food Composition in Japan. The amount of dietary fiber can be measured by the modified Prosky method described in the Standard Tables of Food Composition in Japan, or by a method combined with HPLC (AOAC Official Methods of Analysis (2002)).

In a preferred embodiment of the present invention, the composition contains 200 mg or more of lactic acid per 100 mL of the composition, and in a more preferred embodiment, 300 mg or more of lactic acid is contained in 100 mL of the composition. Lactic acid can be quantified by methods well known to those skilled in the art, such as an enzyme method or HPLC method. Alternatively, the quantification can be carried out referring to the following literature describing methods for quantifying organic acids: J. Nutr., Jan, 124(1), 52-60, 1994 "Galactosylsucrose and xylosylfructoside alter digestive tract size and concentrations of cecal organic acids in rats fed diets containing cholesterol and cholic acid".

Compositions of the present invention are prepared using fermented dairy products. The term "fermented dairy products" used herein generally refers to products prepared by using starters such as lactobacilli to ferment liquid milk preparations by combining one, two, or more kinds of milk sources such as: livestock milk including cow milk, buffalo milk, goat milk, sheep milk, horse milk; and/or partially skimmed milk, skimmed milk, reduced whole milk, reduced skimmed milk, reduced partially skimmed milk, butter, and cream prepared from these livestock milk. For example, natural cheese, yogurt, fermented milk, whey, and whey cheese are included in the fermented dairy products of the present invention. "Cheese" in the present invention refers to that produced by fermenting milk, butter milk, or cream by lactobacilli, or by removing whey from curdled milk produced by adding enzymes to milk, butter milk, or cream. The cheese may or may not be solidified or aged (ripened). *Lactobacillus bulgaricus* and *Streptococcus thermophilus* are the major starters used for manufacturing fermented dairy products but are not limited thereto, and lactobacilli and bifidobacteria such as *Streptococcus lactis, Streptococcus cremoris, Streptococcus diacetilactis, Enterococcus faecium, Enterococcus fecalis, Lactobacillus casei, Lactobacillus helveticus, Lactobacillus acidophilus, Lactobacillus rhamnosis, Lactobacillus plantarum, Lactobacillus murinus, Lactobacillus reuteri, Lactobacillus brevis, Lactobacillus gasseri, Bifidobacterium longum, Bifidobacterium bifidum, Bifidobacterium breve* may be used. Other bacteria, such as *Propionibacterium* which are used to produce fermented dairy products, can also be used in combination with the above. A composition of the present invention can be prepared using any fermented dairy product. Preferably, it is prepared by using unripened cheese or yogurt, and in a more preferred embodiment, it is prepared using quark or yogurt.

Quark is a type of unripened (fresh) cheese low in fat content, and is characterized by a refreshing flavor and an acidic taste. A conventional method for producing quark is as follows: Skimmed milk is sterilized. To this, 0.5% to 5% of a starter lactobacillus (mainly *Lactobacillus bulgaricus* or *Streptococcus thermophilus*) is inoculated for fermentation. A curd is formed when the pH reaches 4.6. This is placed in a quark separator to separate the whey, and the obtained curd is cooled. An example of a composition of quark is 17% to 19% total solids, 11% to 13% proteins, <1% fats, 2% to 8% carbohydrates, and <2% lactose. In addition, quark solidified using rennet are also included in the quark of the present invention. Alternatively, quark can be produced by adding a mixed culture of *lactis* subsp. *lactis,* and *lactis* subsp. *cremoris* belonging to the genus *Lactococcus,* and bacterial strains from the genus *Leuconostoc* to skimmed milk, culturing it, and then removing the whey. Unripened cheese obtained by cutting the curds produced by methods similar to those described above and then separating the whey by heating, may also be used in the present invention.

Compositions of the present invention are prepared as a homogeneous mixture by mixing the fermented dairy product and the above-mentioned carbohydrates, proteins, and fats, such that the percentage of the fermented dairy product-derived protein in the proteins of the composition is preferably 30% or more by weight, or more preferably 70% or more by weight. When a composition of the present invention is made into a liquid diet it is preferably prepared such that 1 mL of the food amounts to 0.7 to 2 kcal. Furthermore, vitamins, minerals, and/or dietary fibers may be added during the mixing process. Dietary fibers are categorized into water-soluble and insoluble dietary fibers and either of them may be used. Examples of water-soluble dietary fibers include pectin (protopectin, pectinic acid, pectic acid), guar gum hydrolysate, glucomannan, galactomannan, psyllium, corn fiber, alginate, alginate degradation products, carrageenan, and indigestible dextrin. Examples of insoluble fibers include crystalline cellulose, beet fiber, and wheat bran. Preferably, pectin, guar gum hydrolysate, and indigestible dextrin may be used. In certain cases, flavoring agents or other compositions may be added.

After mixing, the mixture can be filled into a container, and if necessary, heat sterilized to produce liquid diets or enteral nutritional formulations. Since the anti-Gram-positive bacterial compositions of the present invention are acidic, heating can be carried out under conditions that are milder than general heating conditions. For example, where neutral liquid diet sterilization requires a 20-40 minute retort sterilization at 120-130°C and a 4-10 second indirect sterilization at 140-150°C, the compositions of the present invention can be sterilized by a 15-30 minute retort sterilization at 80-90°C and a 20-60 second indirect sterilization at 95-110°C. Mild sterilization conditions allow flavor improvement as well as the blending of components that are affected by heat. The compositions of the present invention can be made into gels using agar or gelatin, into granular foods/pharmaceuticals by spray drying and such, or into solid foods/pharmaceuticals.

Compositions of the present invention have antibacterial effects on both Gram-negative and Gram-positive bacteria. An excellent effect is exerted particularly against Gram-positive bacteria. While the antibacterial action of lactobacilli-fermented products are generally known, compositions of the present invention have an excellent antibacterial effect on Gram-positive bacteria as compared to the original fermented dairy product(s) used as source. Such excellent antibacterial effects on Gram-positive bacteria are obtained as a result of producing compositions of the present invention by combining the fermented dairy product(s) with carbohydrates, proteins, fats, and such. The fact that such effects can be obtained by processing the fermented dairy product(s) into compositions of the present invention was first discovered by the present inventors. The present inventors confirmed that the above-mentioned effects are observed against *Staphylococcus aureus* subsp. *aureus,* MRSA, *Streptococcus mutans,* and *Clostridium difficile,* as described in the examples. Antibacterial activities against other Gram-positive bacteria, such as those of the genus *Streptococcus, Enterococcus, Corynebacterium, Bacillus,* and C. *botulinum,* can also be expected. Compositions of the present invention comprise the above-mentioned antibacterial activities, and when the antibacterial action against *Staphylococcus aureus* subsp. *aureus,* MRSA, *Streptococcus mutans,* and *Clostridium difficile* is measured by the method described in the examples, the viable bacterial cell count after incubation for 24 hours is preferably $2.3 \times 10^2$ CFU (colony forming unit)/mL or less, and most preferably less than 10 colonies after 24hours of incubation.

Compositions of the present invention may be used as liquid diets or as enteral nutritional agents that have antibacterial effects, especially anti-Gram-positive bacterial effects. Since the liquid diets or the enteral nutritional agents themselves contain antibacterial effects, they may be not only used as foods but can be used for the purpose of preventing infections. They may be used as Food with Health Claims, such as Food for Specified Health Uses, and Food with Nutrient Function Claims, which are foods with additional properties such as being able to prevent infections, having antibacterial activities, or having excellent antibacterial activities against Gram-positive bacteria. At institutions which accommodate a large number of elderly people or hospitalized patients, such food may provide effective and safe preventive measures against hospital-acquired infection/institutional infections. They can also be used as antibacterial baby food for infants, or as antibacterial nutritional foods for general use. They may further be used as nutritional supplementary pharmaceuticals for controlling intestinal functions, or foods for preventing tooth decay.

Examples

The present invention is explained in detail below with reference to examples, but it is not to be construed as being limited thereto.

[Example 1] Examination of an acidic liquid diet's antibacterial effect (1)

An acidic liquid diet's antibacterial effect against pathogenic bacteria was examined. *Escherichia coli* IFO 3972, *Staphylococcus aureus* subsp. *aureus* IFO 12732, *Pseudomonas aeruginosa* NBRC 13275, *Staphylococcus aureus* 11D 1677 (MRSA), and *Streptococcus mutans* IFO 13955 were used as the bacteria examined. *Streptococcus mutans* was cultured using Trypto Soy agar medium (Eiken Chemical) and the other test bacteria were cultured using a regular agar medium (Eiken Chemical). Subsequently, bacterial suspensions were prepared by suspending the obtained bacterial cells in a sterilized physiological saline solution to make the number of bacterial cells per 1 mL of solution approximately $10^8$-$10^9$. The composition described in Table 1 was used to prepare the acid acidic liquid diet. A neutral liquid diet having the composition indicated in Table 2 (Meibalance, Meiji Dairies) and a liquid fermented milk preparation were used as control samples. The liquid fermented milk preparation was prepared by adding sterile distilled water to quark (a fermented dairy product) such that the concentration of quark (33 g/100 mL) was the same in the acidic liquid diet prepared according to the composition shown in Table 1. This was used after killing the lactobacilli by heating at 100°C for 10 minutes. The method for producing quark was as follows: First, skimmed milk was inoculated with 1% of a lactobacilli starter (a combined starter of *Lactobacillus bulgaricus* and *Streptococcus thermophilus*), and this was fermented at 35°C for 16 hours. The obtained curd was placed in a quark separator, and quark containing approximately 13% proteins, approximately 0.3% fats, approximately 5% carbohydrates, and approximately 19% total solids was obtained by separating out the whey. Mixed oils and fats comprise fatty acids such as palmitic acid, oleic acid, linoleic acid, linolenic acid, eicosapentaenoic acid, and docosahexaenoic acid. Within the fatty acid composition, the ratio of fatty acids comprising double bonds is 25%, and the ratio of n-6/n-3 is 7.4%.

Table 1

| Ingredients | Blended Quantity (per 100 mL) |
| --- | --- |
| Fermented dairy product | 33.4 g |
| Honey | 8 g |
| Dextrin | 6.1 g |
| Sucrose | 1 g |
| Indigestible dextrin | 0.61 g |
| Pectin | 0.75 g |
| Mixed oils and fats | 2.6 g |
| Soybean lecithin | 0.13 g |

1 mL of *Escherichia coli, Pseudomonas aeruginosa, Staphylococcus aureus* subsp. *aureus,* MRSA, or *Streptococcus mutans* was added to 100 mL of the acidic liquid diet or controls, and cultured at 37°C. After 3, 6, and 24 hours of culturing, the cells were sampled, and cultured on an agar plate and the viable bacterial cell count was determined as follows: samples were appropriately diluted with SCDLP medium (Nihon Pharmaceutical), cultured at 35°C for 2 days by pour-plate techniques using SCDLPA culture medium (Nihon Pharmaceutical) after which the number of developing colonies were counted. Sterilized physiological saline solution was used as a control.

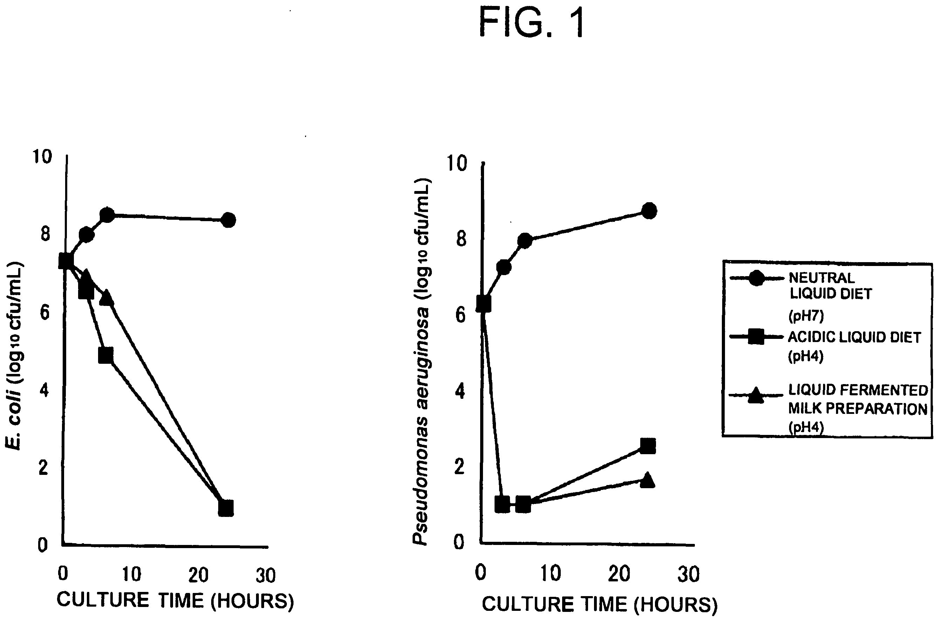

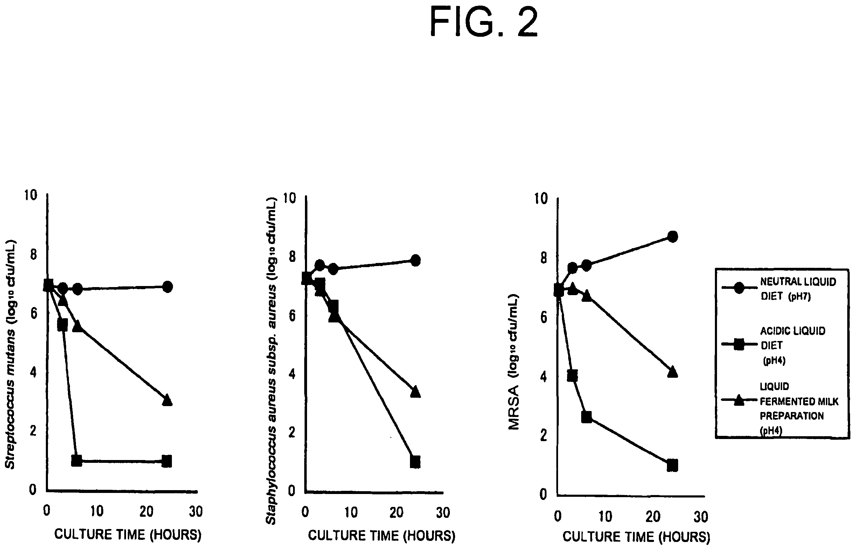

The results are shown in Figs. 1 and 2. The acidic liquid diet showed a suppressive effect on the growth of each bacterial strain, and furthermore the antibacterial effect on each bacterial strain presented unexpected but interesting results. The viable bacterial cell count (number of colonies) of *E. coli* cultured in the liquid fermented milk preparation was 20,000,000 on initial culturing, 8,000,000 after a 3-hour culturing, 2,500,000 after a 6-hour culturing, and below the detection limit after 24-hour culturing. On the other hand, the viable cell count of *E. coli* cultured in the acidic liquid diet was 20,000,000 on the initial culturing, 3,400,000 after a 3-hour culturing, 83,000 after a 6-hour culturing, and below the detection limit after 24-hour culturing (In the present example, "below the detection limit" means that the viable cell count is less than 10. This term has the same meaning hereinafter.). The viable bacterial cell count (number of colonies) of *Pseudomonas aeruginosa* cultured in the liquid fermented milk preparation was 2,000,000 on initial culturing, and below the detection limit after a 3-hour and 6-hour culturing, whereas the viable bacterial cell count of *Pseudomonas aeruginosa* cultured in the acidic liquid diet was 2,000,000 on the initial culturing, and below the detection limit after a 3-hour and 6- hour culturing. That is, the growth suppressive effect of the acidic liquid diet on *E. coli* and *Pseudomonas aeruginosa,* which are Gram-negative bacteria, was at the same level as that of the liquid fermented milk preparation. Therefore, the antibacterial effect of the acidic liquid diet on each of the above-mentioned bacteria is considered to be an effect caused mainly by components derived from the fermented dairy product. In the neutral liquid diet, growth of each type of bacteria was promoted along with culture time (Fig. 1). Antibacterial effect was examined in other commercially available neutral liquid diets, but as in Meibalance, no antibacterial effect was observed (data not shown). On the other hand, results of Gram-positive bacteria *Staphylococcus aureus* subsp. *aureus,* MRSA, and *Streptococcus mutans,* showed that the growth suppressive effect of the acidic liquid diet is remarkably higher than that of the liquid fermented milk preparation (Fig. 2). The viable cell count (number of colonies) of *Staphylococcus aureus* subsp. *aureus* cultured in the liquid fermented milk preparation was 18,000,000 on initial culturing, 7,500,000 after a 3-hour culturing, and 1,000,000 after a 6-hour culturing; whereas, the viable bacterial cell count of *Staphylococcus aureus* subsp. *aureus* cultured in the acidic liquid diet was 18,000,000 on initial culturing, 12,000,000 after a 3-hour culturing, 2,100,000 after a 6-hour culturing, and below the detection limit after 24-hour culturing. The viable bacterial cell count (number of colonies) of *Streptococcus mutans* cultured in the liquid fermented milk preparation was 9,000,000 on initial culturing, 2,900,000 after a 3-hour culturing, 400,000 after a 6 -hour culturing, and 1,300 after a 24-hour culturing; whereas, the viable cell count of *Streptococcus mutans* cultured in the acidic liquid diet was 9,000,000 on initial culturing, 430,000 after a 3-hour culturing, and already below the detection limit at 6 hours of culturing. As described, the acidic liquid diet not only has antibacterial effects against Gram-negative bacteria and Gram-positive bacteria, but also has higher antibacterial effect, especially against Gram-positive bacteria, which far exceeds the antibacterial effect of the fermented dairy product, which is the original source.

[Example 2] Examination of antibacterial effect of organic acids

Since the antibacterial effect of the acidic liquid diet was verified, the composition of the acidic liquid diet was next investigated. The composition of the neutral liquid diet, acidic liquid diet, and liquid fermented milk preparation are shown in Table 2. The neutral liquid diet contains citric acid for pH adjustment, but hardly contains any other organic acids. The liquid fermented milk preparation contained 405.1 mg/100 mL of lactic acid produced by lactic acid fermentation. The acidic liquid diet contained approximately twice the amount of lactic acid than that of the liquid fermented milk preparation; however, this is due to the addition of a supplemental calcium lactate to secure the calcium level required in liquid diets. In addition to lactic acid, the acidic liquid diet and liquid fermented milk preparation abundantly contain citric acid and acetic acid as major organic acids (Table 2).

Table 2

| | Components | Neutral liquid diet | Acidic liquid diet | Liquid fermented milk preparation | Units (per 100 mL) |
|---|---|---|---|---|---|
| General composition | Proteins | 4.0 | 4.0 | 4.0 | g |
| | Fats | 2.8 | 2.8 | 0.0 | g |
| | Carbohydrates | 15.7 | 16.1 | 1.6 | g |
| | Ash | 0.8 | 0.7 | 0.3 | g |
| | Calories | 100.0 | 100.0 | 22.4 | Kcal |

(continued)

| | Components | Neutral liquid diet | Acidic liquid diet | Liquid fermented milk preparation | Units (per 100 mL) |
|---|---|---|---|---|---|
| Organic acids | Lactic acid | 0.0 | 838.8 | 405.1 | mg |
| | Acetic acid | 1.3 | 8.5 | 5.3 | mg |
| | Citric acid | 126.2 | 172.9 | 64.0 | mg |
| | Formic acid | 1.9 | 1.9 | 0.4 | mg |

The antibacterial effects of major organic acids contained in the acidic liquid diet and in the fermented dairy product were examined. Preparation of testing bacteria and bacterial cultures was carried out similarly to as described above when antibacterial effect of the acidic liquid diet was examined. As a test sample, any one of 1 mL of lactic acid, 2.3 mL of acetic acid, or 1 g of citric acid (Wako Pure Chemical) was added to the neutral liquid diet, and the pH adjusted to 4. Antibacterial effect of lactic acid, the major organic acid of the acidic liquid diet, was examined using samples prepared by adding varying amounts of lactic acid to the neutral liquid diets.

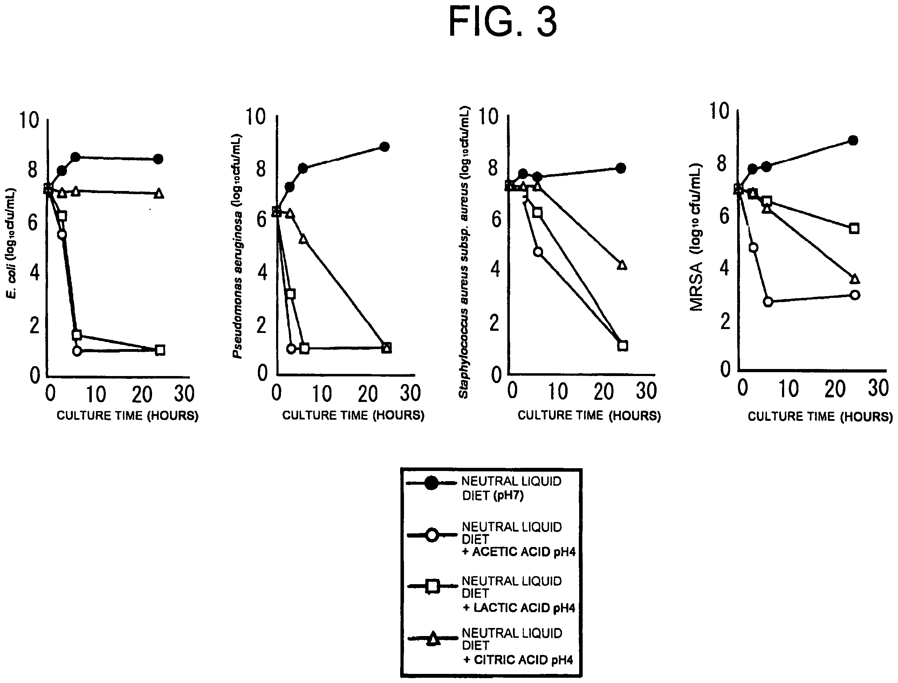

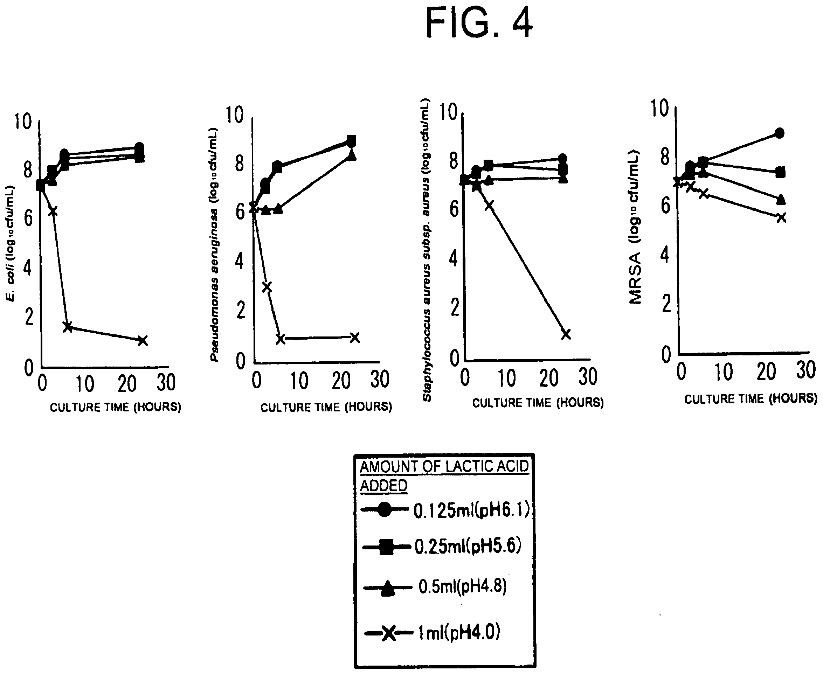

A test sample of pH 5.1, which was prepared by adding, to the neutral liquid diet, an amount of organic acid equivalent to the amount contained in the liquid fermented milk preparation shown in Table 2 (lactic acid: 405.1 mg; acetic acid: 5.3 mg; citric acid: 34 mg; and formic acid: 0.4 mg) hardly exhibited any antibacterial effect against any of the bacterial species. Samples prepared by adding lactic acid and acetic acid independently to the neutral liquid diet and adjusting the pH to 4 showed an antibacterial effect against *Escherichia coli, Pseudomonas aeruginosa, Staphylococcus aureus* subsp. *aureus,* and MRSA (Fig. 3). The antibacterial effect of the citric acid-supplemented sample against these four bacterial species was weaker compared to those activities of lactic acid or acetic acid-supplemented samples at the same pH (Fig. 3). Meanwhile, when the neutral liquid diet samples were supplemented only with 0.5 mL of lactic acid (pH4.8), which is the major organic acid in the acidic liquid diet and the liquid fermented milk preparation, hardly any antibacterial effect was seen, whereas a notable antibacterial effect against *Escherichia coli, Pseudomonas aeruginosa,* and *Staphylococcus aureus* subsp. *aureus* was observed (Fig. 4) when the samples were adjusted to pH4,.

A distinct tendency was observed regarding antibacterial effect against MRSA. More specifically, when the antibacterial effects of each of the respective organic acids against MRSA were compared, acetic acid was the strongest, citric acid was next, followed by lactic acid. The antibacterial effect of lactic acid, which is the major organic acid in the fermented dairy product, was weakest amongst the examined organic acids. Since the antibacterial effect of the acidic liquid diet against MRSA was prominent compared to the liquid fermented milk preparation, the antibacterial effect against MRSA of the acidic liquid diet is thought to involve component(s) other than the fermented dairy product components.

[Example 3] Examination of antibacterial effect of the acidic liquid diet (2)

The antibacterial effect of the acidic liquid diet against various bacteria was confirmed as mentioned above. In addition, the antibacterial effect against *Helicobacter pylori* ATCC43504 and *Clostridium difficile* JCM1296 was examined. *Helicobacter pylori* is a Gram-negative bacteria and *Clostridium difficile* is a Gram-positive bacteria. Antibacterial effect against *Helicobacter pylori* was examined as follows: First, the bacterial cells were cultured on a sheep blood agar medium (Eiken Chemical) under a microaerophilic atmosphere at 35°C for 3 days, after which the cells were suspended in a sterilized physiological saline solution such that the number of bacterial cells in the prepared suspension was approximately $10^7$ to $10^8$ cells per 1 mL. 0.5 mL of the *Helicobacter pylori* suspension was added to 4.5 mL of the acidic liquid diet, and to control samples of sterilized physiological saline solution and 4.5 mL of Brucella broth supplemented with 5% horse serum. Then the cells were cultured under a microaerophilic atmosphere at 35°C. After culturing for 3, 6, and 24 hours, cultures were sampled, and the viable bacterial cell count was determined as follows: The samples were appropriately diluted with a sterilized physiological saline solution, and then swiped onto a sheep blood agar plate (Eiken Chemical). After culturing under a microaerophilic atmosphere at 35°C for 5 days, the formed colony number was counted. Additionally, antibacterial effect against *Clostridium difficile* was examined as follows: *Clostridium difficile* cells were cultured in a GAM agar medium (Nihon Pharmaceutical) under anaerobic conditions at 37°C for 15 days, and then the cells were suspended in a sterilized physiological saline solution such that the number of bacterial cells in the prepared suspension was approximately $10^8$ to $10^9$ cells per 1 mL. 1 mL of the suspension was added to 100 mL of the acidic liquid diet samples and to the controls, and the cells were cultured under anaerobic conditions at 37°C. After culturing for 3, 6, and 24 hours, the cultures were sampled, and the viable bacterial cell count (number of anaerobic bacterial cells) was determined. The number of viable *Clostridium difficile* cells was determined out as follows. The samples were appropriately diluted with SCDLP medium (Nihon Pharmaceutical), and the number of anaerobic bacterial cells was determined by a pour-plate technique using a GAM agar plate (Nihon Pharmaceutical) on which the cells were cultured under anaerobic conditions at 35°C for 5 to 6 days, after which the formed colony number was counted.

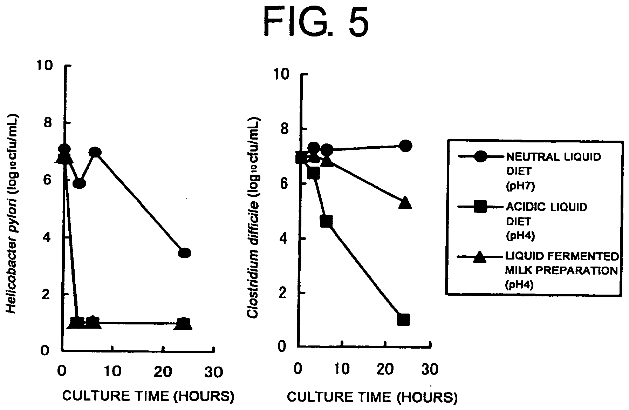

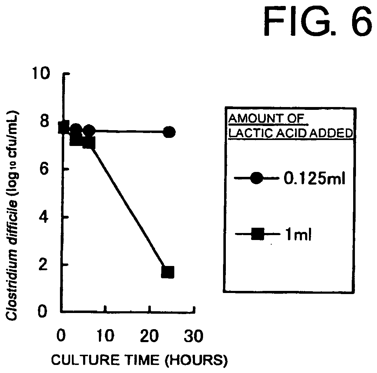

The results are shown in Fig. 5. The acidic liquid diet showed an excellent antibacterial action against *Helicobacter pylori,* a Gram-negative bacterium, and the degree of antibacterial effect matched that of the liquid fermented milk preparation. On the other hand, the acidic liquid diet showed a much higher antibacterial action against *Clostridium difficile,* a Gram-positive bacterium, as compared to that of the liquid fermented milk preparation. These results demonstrated the same tendency as in the results obtained in the above examples. Furthermore, when lactic acid was added to the neutral liquid diet to examine the antibacterial action against *Clostridium difficile,* as in Example 2, addition of 0.125 mL lactic acid (pH6.1) hardly showed any antibacterial action, while an antibacterial effect was observed with addition of 1 mL lactic acid (Fig. 6).

[Example 4] Examination of antibacterial action in a different acidic liquid diet composition

Another acidic liquid diet (hereinafter referred to as "acidic liquid diet 2") with a composition different to the above-mentioned acidic liquid diet was prepared based on the blend shown in Table 3, and the antibacterial action against Gram-positive bacteria was examined. The composition of acidic liquid diet 2 is shown in Table 4.

Table 3

| Ingredients | Blended Quantity (per 100 mL) |
|---|---|
| Fermented dairy product | 22.7 g |
| Whey protein hydrolysate | 1.42 g |
| Palatinose | 5.6 g |
| Dextrin | 5.2 g |
| Maltodextrin | 1.9 g |
| Indigestible dextrin | 1.04 g |
| Pectin | 0.45 g |
| Mixed oils and fats | 3.0 g |
| Phospholipids | 0.1 g |
| Soybean lecithin | 0.16 g |

Table 4

| | Components | Acidic liquid diet 2 | Units (per 100 mL) |
|---|---|---|---|
| General composition | Proteins | 4.2 | g |
| | Fats | 3.0 | g |
| | Carbohydrates | 14.1 | g |
| | Ash | 0.8 | g |
| | Calories | 100.0 | Kcal |
| Organic acids | Lactic acid | 405.1 | mg |
| | Acetic acid | 5.3 | mg |
| | Citric acid | 388.0 | mg |
| | Formic acid | 1.9 | mg |

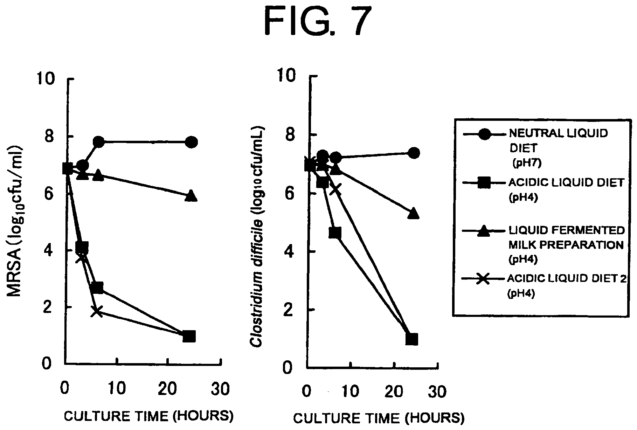

Acidic liquid diet 2 was tested using the same method as that used for the above-mentioned acidic liquid diet. The viable bacterial cell count of MRSA was 7,700,000 on initial culturing, already 5,600 after a 3-hour culturing, 70 after a 6-hour culturing, and below the detection limit after a 24- hour culturing, whereas the viable bacterial cell count of MRSA incubated in the liquid fermented milk preparation was 7,700,000 on initial culturing, 4,900,000 after a 3-hour culturing, 4,600,000 after a 6-hour culturing, and 920,000 after a 24-hour culturing. The viable bacterial cell count of *Clostridium difficile* cultured in the liquid fermented milk preparation was 9,100,000 on initial culturing, 10,000,000 after a 3-hour culturing, 7,000,000 after a 6-hour culturing, and 220,000 after a 24-hour culturing; whereas, the viable bacterial cell count of *Clostridium difficile* cultured in acidic liquid diets 2 decreased drastically from 12,000,000 on initial culturing to 9,300,000 after a 3-hour culturing, to 1,400,000 after a 6-hour culturing, and finally to below the detection limit after a 24-hour culturing. The viable bacterial cell count of *Clostridium difficile* cultured in the acidic liquid diet of Example 1 also drastically decreased from 9,100,000 on initial culturing to 2,400,000 after a 3-hour culturing, to 44,000 after a 6-hour culturing, and finally to below the detection limit after a 24-hour culturing. As described above, the tendency and levels of antibacterial action of acidic liquid diet 2 against MRSA and *Clostridium difficile* almost matched those of the above-mentioned acidic liquid diets examined in Examples 1 and 2 (Fig. 7).

Another acidic liquid diet, acidic liquid diet 3, was prepared according to the blend indicated in Table 5. Energy ratios of the acidic liquid diet, acidic liquid diet 2, and acidic liquid diet 3 of the present example are shown in Table 6.

Table 5

| Ingredients | Blended Quantity (per 100 mL) |
|---|---|
| Fermented dairy product | 15.3 g |
| Honey | 7.5 g |
| Dextrin | 16 g |
| Sucrose | 1.5 g |
| Indigestible dextrin | 0.61 g |
| Pectin | 0.75 g |
| Adjusted oils and fats | 2.6 g |
| Soybean lecithin | 0.13 g |

Table 6

| | Components | Acidic liquid diet | Acidic liquid diet 2 | Acidic liquid diet 3 | Units |
|---|---|---|---|---|---|
| General composition | Proteins | 16 | 17 | 7 | % |
| | Fats | 24 | 27 | 24 | % |
| | Carbohydrates | 58 | 55 | 67 | % |
| Organic acids | Lactic acid | 838.8 | 405.1 | 188.4 | mg |
| | Acetic acid | 8.5 | 5.3 | 2.5 | mg |
| | Citric acid | 172.9 | 388.0 | 380 | mg |
| | Formic acid | 1.9 | 1.9 | 0.2 | mg |

Industrial Applicability

The present invention provides novel antibacterial compositions. The compositions of the present invention are highly effective particularly against Gram-positive bacteria. Other than as antibiotics, the compositions of the present invention may be used as a means to protect easily infected individuals, such as the elderly, against infection by using liquid diets or enteral nutritional formulations. Since the liquid diets themselves have an antibacterial effect, such methods can be convenient and effective for preventing institutional infections that occur via catheters and such. Anti-Gram-positive-bacteria compositions of the present invention are highly practical considering that Gram-positive bacterial species such as MRSA, which is a representative causative bacterium of opportunistic infections, are important in terms of hygiene. Furthermore, since the compositions of the present invention comprise fermented dairy products, they are expected to exhibit the following effects: suppressive effects on adhesion of pathogenic bacteria to the intestinal epithelium; therapeutic/prophylactic effects on infection due to production of antibacterial substances; stimulative effects on host immunogenic potential through macrophage activation and maintenance of a healthy intestinal environment; and probiotic effects of lactobacilli in preventing diarrhea/constipation. The compositions of the present invention can be used to produce orally administered antibacterial foods or pharmaceuticals, and are very useful for nutrition and maintenance of health and hygiene.

## Claims

1. An antibacterial composition having an activity of inhibiting the proliferation of gram-positive bacteria, which comprises the constituent combination of any one of (a) to (c):

   (a) 33.4 g cheese, 8 g honey, 6.1 g dextrin, 1 g sucrose, 0.61 g indigestible dextrin, 0.75 g pectin, 2.6 g mixed oils and fats, and 0.13 g soybean lecithin, per 100 ml composition;
   (b) 22.7 g cheese, 1.42 g whey protein hydrolysate, 5.6 g palatinose, 5.2 g dextrin, 1.9 g maltodextrin, 1.04 g indigestible dextrin, 0.45 g pectin, 3.0 g mixed oils and fats, 0.1 g phospholipids, and 0.16 g soybean lecithin, per 100 ml composition; and
   (c) 15.3 g cheese, 7.5 g honey, 16 g dextrin, 1.5 g sucrose, 0.61 g indigestible dextrin, 0.75 g pectin, 2.6 g mixed oils and fats, and 0.13 g soybean lecithin, per 100 ml composition.

2. A method for producing an antibacterial composition, wherein the method comprises mixing the cheese with edible carbohydrates, proteins, and fats, and then homogenizing and sterilizing the mixture, and wherein the composition

   a) is prepared using a cheese,
   b) comprises edible carbohydrates, proteins, and fats,
   c) has a pH of 4.6 or less,
   d) has an activity of inhibiting the proliferation of bacteria, and
   e) is prepared as a homogeneous mixture,

   wherein the carbohydrate energy ratio is from 50 % to 70 % and calculated as

   $$\text{carbohydrate energy ratio} = \text{carbohydrate amount (g)} \times 16.74 \text{ kJ/g (4 kcal/g)} \div \text{total food energy (kJ)(kcal)} \times 100, \text{ or}$$

   $$\text{carbohydrate energy ratio} = \{\text{total food energy ((kJ)(kcal)} - (\text{protein energy (kJ)(kcal)} + \text{fat energy (kJ) (kcal)} + \text{dietary fiber energy (kJ)(kcal)})\} \div \text{total food energy (kJ)(kcal)} \times 100,$$

   wherein the protein energy ratio is from 4 % to 25 % and calculated as

   $$\text{protein energy ratio} = \text{protein amount (g)} \times 16.74 \text{ kJ/g (4 kcal/g)} \div \text{total food energy (kJ)(kcal)} \times 100, \text{ and}$$

   wherein the fat energy ratio is from 20 % to 30 % and calculated as

   $$\text{fat energy ratio} = \text{fat amount (g)} \times 37.66 \text{ kJ/g (9 kcal/g)} \div \text{total food energy (kJ)(kcal)} \times 100,$$

   and wherein the proteins derived from cheese account for 30 weight % or more of the proteins in the composition.

3. The method of claim 2 for producing the antibacterial composition wherein the cheese is a quark.

4. The method of claim 2 or 3 for producing the antibacterial composition wherein the cheese is prepared by fermenting with a lactobacillus starter.

5. The method of any one of claims 2 to 4 for producing the antibacterial composition wherein the method comprises preparing the composition such that the proteins derived from the cheese account for 70 weight % or more of the proteins in the composition.

6. The method of any one of claims 2 to 5 for producing the antibacterial composition, wherein the composition has an activity of inhibiting the proliferation of gram-positive bacteria.

**Patentansprüche**

1. Antibakterielle Zusammensetzung mit einer hemmenden Wirkung auf die Proliferation von grampositiven Bakterien, welche eine Kombination von Bestandteilen von irgendeinem aus

   (a) bis (c) umfasst:

   (a) 33,4 g Käse, 8 g Honig, 6,1 g Dextrin, 1 g Saccharose, 0,61 g unverdauliches Dextrin, 0,75 g Pektin, 2,6 g gemischte Öle und Fette, und 0,13 g Sojalecithin, pro 100 ml Zusammensetzung;
   (b) 22,7 g Käse, 1,42 g Molkenprotein-Hydrolysat, 5,6 g Palatinose, 5,2 g Dextrin, 1,9 g Maltodextrin, 1,04 g unverdauliches Dextrin, 0,45 g Pektin, 3,0 g gemischte Öle und Fette, 0,1 g Phospholipide und 0,16 g Sojalecithin, pro 100 ml Zusammensetzung; und
   (c) 15,3 g Käse, 7,5 g Honig, 16 g Dextrin, 1,5 g Saccharose, 0,61 g unverdauliches Dextrin, 0,75 g Pektin, 2,6 g gemischte Öle und Fette, und 0,13 g Sojalecithin, pro 100 ml Zusammensetzung.

2. Verfahren zur Herstellung einer antibakteriellen Zusammensetzung, wobei das Verfahren das Mischen des Käses mit essbaren Kohlenhydraten, Proteinen und Fetten, und das anschließende Homogenisieren und Sterilisieren des Gemisches umfasst, und wobei die Zusammensetzung

   a) unter Verwendung eines Käses hergestellt wird,
   b) essbare Kohlenhydrate, Proteine und Fette enthält,
   c) einen pH-Wert von 4,6 oder weniger aufweist,
   d) eine hemmende Wirkung auf die Proliferation von Bakterien aufweist, und
   e) als eine homogene Mischung hergestellt wird,

   wobei das Kohlenhydrat-Energieverhältnis 50% bis 70% beträgt und berechnet wird als

   Kohlenhydrat-Energieverhältnis = Kohlenhydratmenge (g) x 16,74 kJ/g (4 kcal/g) ÷ gesamte Nahrungsenergie (kJ) (kcal) x 100, oder

   Kohlenhydrat-Energieverhältnis = {gesamte Nahrungsenergie ((kJ) (kcal) - (Proteinenergie (kJ) (kcal) + Fettenergie (kJ) (kcal) + Ballaststoffenergie (kJ) (kcal))} ÷ gesamte Nahrungsenergie (kJ) (kcal) x 100,

   wobei das Protein-Energieverhältnis 4% bis 25% beträgt und berechnet wird als

   Protein-Energieverhältnis = Proteinmenge (g) x 16,74 kJ/g (4 kcal/g) ÷ gesamte Nahrungsenergie (kJ) (kcal) x 100, und

   wobei das Fett-Energieverhältnis 20% bis 30% beträgt und berechnet wird als

   Fett-Energieverhältnis = Fettmenge (g) x 37,66 kJ/g (9 kcal/g) ÷ gesamte Nahrungsenergie (kJ) (kcal) x 100,

   und wobei die aus Käse abgeleiteten Proteine 30 Gewichts-% oder mehr der Proteine in der Zusammensetzung ausmachen.

**3.** Verfahren nach Anspruch 2 zur Herstellung der antibakteriellen Zusammensetzung, wobei der Käse ein Quark ist.

**4.** Verfahren nach Anspruch 2 oder 3 zur Herstellung der antibakteriellen Zusammensetzung, wobei der Käse durch Fermentieren mit einem Lactobacillus-Starter hergestellt wird.

**5.** Verfahren nach einem der Ansprüche 2 bis 4 zur Herstellung der antibakteriellen Zusammensetzung, wobei das Verfahren eine solche Herstellung der Zusammensetzung umfasst, dass die aus dem Käse abgeleiteten Proteine 70 Gewichts-% oder mehr der Proteine in der Zusammensetzung ausmachen.

**6.** Verfahren nach einem der Ansprüche 2 bis 5 zur Herstellung der antibakteriellen Zusammensetzung, wobei die Zusammensetzung eine hemmende Wirkung auf die Proliferation von grampositiven Bakterien aufweist.


**Revendications**

**1.** Composition antibactérienne présentant une activité d'inhibition de la prolifération de bactéries à Gram positif, qui comprend la combinaison de constituants de n'importe lesquels de (a) à (c) :

(a) 33,4 g de fromage, 8 g de miel, 6,1 g de dextrine, 1 g de saccharose, 0,61 g de dextrine non digestible, 0,75 g de pectine, 2,6 g d'huiles et de matières grasses mixtes, et 0,13 g de lécithine de soja, pour 100 ml de composition ;
(b) 22,7 g de fromage, 1,42 g d'hydrolysat de protéine de lactosérum, 5,6 g de palatinose, 5,2 g de dextrine, 1,9 g de maltodextrine, 1,04 g de dextrine non digestible, 0,45 g de pectine, 3,0 g d'huiles et de matières grasses mixtes, 0,1 g de phospholipides, et 0,16 g de lécithine de soja, pour 100 ml de composition ; et
(c) 15,3 g de fromage, 7,5 g de miel, 16 g de dextrine, 1,5 g de saccharose, 0,61 g de dextrine non digestible, 0,75 g de pectine, 2,6 g d'huiles et de matières grasses mixtes, et 0,13 g de lécithine de soja, pour 100 ml de composition.

**2.** Procédé de production d'une composition antibactérienne, le procédé comprenant le mélange du fromage avec des hydrates de carbone, des protéines, et des matières grasses comestibles, et ensuite l'homogénéisation et la stérilisation du mélange, et la composition

a) étant préparée en utilisant un fromage,
b) comprend des hydrates de carbone, des protéines, et des matières grasses comestibles,
c) ayant un pH de 4,6 ou moins,
d) ayant une activité d'inhibition de la prolifération des bactéries, et
e) étant préparée sous la forme d'un mélange homogène,

le rapport de la valeur énergétique des hydrates de carbone étant de 50 % à 70 % et calculé par

rapport de la valeur énergétique des hydrates de carbone = quantité d'hydrate de carbone (g) x 16,74 kJ/g (4 kcal/g) ÷ valeur énergétique totale des aliments (kJ)(kcal) x 100, ou

rapport de la valeur énergétique des hydrates de carbone = {valeur énergétique totale des aliments ((kJ)(kcal) - (valeur énergétique des protéines (kJ)(kcal) + valeur énergétique des matières grasses (kJ)(kcal) + valeur énergétique des fibres alimentaires (kJ)(kcal))} ÷ valeur énergétique totale des aliments (kJ)(kcal) x 100,

le rapport de la valeur énergétique des protéines étant de 4 % à 25 % et calculé par

rapport de la valeur énergétique des protéines = quantité de protéine (g) x 16,74 kJ/g (4 kcal/g) ÷ valeur énergétique totale des aliments (kJ)(kcal) x 100, et

le rapport de la valeur énergétique des matières grasses étant de 20 % à 30 % et calculé par

rapport de la valeur énergétique des matières grasses = quantité de matière grasse (g) x 37,66 kJ/g (9 kcal/g) ÷ valeur énergétique totale des aliments (kJ)(kcal) x 100,

et les protéines dérivées du quark comptent pour 30 % en poids ou plus des protéines dans la composition.

3. Procédé selon la revendication 2 de production de la composition antibactérienne, le fromage étant du quark.

4. Procédé selon la revendication 2 ou 3 de production de la composition antibactérienne, le fromage étant préparé par fermentation avec un activateur lactobacillus.

5. Procédé selon l'une quelconque des revendications 2 à 4 pour la production de la composition antibactérienne, le procédé comprenant la préparation de la composition de sorte que les protéines dérivées du fromage comptent pour 70 % en poids ou plus des protéines dans la composition.

6. Procédé selon l'une quelconque des revendications 2 à 5 pour la production de la composition antibactérienne, la composition ayant une activité d'inhibition de la prolifération des bactéries à Gram positif.

# FIG. 1

FIG. 2

FIG. 3

# FIG. 4

AMOUNT OF LACTIC ACID ADDED
- ●— 0.125ml(pH6.1)
- ■— 0.25ml(pH5.6)
- ▲— 0.5ml(pH4.8)
- ✕— 1ml(pH4.0)

EP 2 436 274 B1

FIG. 5

FIG. 6

EP 2 436 274 B1

# FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GOEPFERT, J. M ; CHUNG, K. C.** Behavior of salmonellae in sliced luncheon meats. *Appl. Microbiol.,* 1970, vol. 19, 190-192
- **NAKAE, E.** *Science of Milk, Meat, and Eggs, Kougaku Shuppan,* 1996, 132-137
- **KAWAI Y ; SAITO, T. ; KITAZAWA, H. ; ITOH T.** *Biosci. Biotech. Biochem.,* 1998, vol. 62, 22438-40

- **SHAH, N. P.** Some beneficial effects of probiotic bacteria. *Bioscience Microflora,* 2000, vol. 19 (2), 99-106
- Galactosylsucrose and xylosylfructoside alter digestive tract size and concentrations of cecal organic acids in rats fed diets containing cholesterol and cholic acid. *J. Nutr.,* January 1994, vol. 124 (1), 52-60